# EUROPEAN PATENT SPECIFICATION

(11) **EP 4 593 893 B1**
(45) Date of publication and mention of the grant of the patent: **08.04.2026**
(21) Application number: 23783187.0
(22) Date of filing: 12.09.2023
(51) Int. Cl.: A61L 2/18, A61L 2/26, A61B 1/12

(54) **RUNOFF SEAL FOR HINGED LID OF MEDICAL DEVICE PROCESSOR**
ABLAUFDICHTUNG FÜR KLAPPDECKEL EINES PROZESSORS MEDIZINISCHER VORRICHTUNGEN
JOINT DE RUISSELLEMENT POUR COUVERCLE ARTICULÉ DE PROCESSEUR DE DISPOSITIF MÉDICAL

(30) Priority: 30.09.2022 US 202263411663 P
(43) Date of publication of application: 06.08.2025
(73) Proprietor: American Sterilizer Company, Mentor, OH 44060-1834 (US)
(72) Inventor: LANGE, Ethan K., Painesville, Ohio 44077 (US)
(74) Representative: FRKelly
(86) International application number: PCT/US2023/032499
(87) International publication number: WO 2024/072623

(56) References cited:
- DE-U1- 202012 100 997
- US-A1- 2005 012 281
- US-A1- 2011 262 301

## Description

### Field of Invention

This application relates generally to a runoff seal for a hinged lid and a method of operating such a runoff seal, and more particularly to a runoff seal for a hinged lid of a medical device processor and a method of operating such a runoff seal.

### Background

Medical device processors, also referred to as medical instrument processors, are widely used in various health care settings and are typically associated with a sterilization claim or a high level disinfection claim. An example of such a processor is an automated endoscope reprocessor (AER) used for reprocessing endoscopes, such as duodenoscopes, and endoscope accessories. AERs are designed to kill microorganisms in or on reusable endoscopes by exposing their outside surfaces and interior channels to liquid chemical sterilant or high level disinfectant solutions.

Processors that have horizontal hinging lids or doors face various challenges in managing fluid runoff from the lid when the lid is hinged open after a processing cycle. At the end of a processing cycle, the lid may have collected fluid droplets, either due to immersion, spray, or condensation. As the lid hinges from a horizontal or near horizontal closed position to a more vertical open position, gravity causes the fluid droplets to run down the lid. It is desirable to prevent such runoff from potentially contacting non-decontaminated surfaces and subsequently the disinfected/sterilized load.

For runoff seals or mechanisms in some medical device processors, there remain various shortcomings, drawbacks, and disadvantages relative to certain applications. For example, one technique is to provide a drainage channel system in the processing tray/basin of the medical device processor. When the lid is opened, the channel will receive the non-decontaminated runoff from the lid and direct the runoff away from the basin and processed load. While this solution has proven effective for sterilant processing systems, the shielding channel requires additional area outside of the sealing interface, which can be a shortcoming when working in space constrained applications. US Patent Publication No. 2011/262301 discloses a cassette for steam sterilizing of instruments comprising a lid and a tray, the tray having an inlet and an outlet for the cassette for communication with a sterilization apparatus, and the lid having a seal configured to interface with the tray to maintain either pressure or vacuum conditions for an interior of the cassette. German Patent Publication No. DE 20 2012 100997 discloses a medical seal for a medical sterilization container. US Patent Publication No. 2005/012281 discloses an inflatable seal member for providing a fluid-tight seal.

Accordingly, there remains a need for further contributions in this area of technology.

### Summary of Invention

The application relates to a medical device processor including a basin, a lid, and a flexible seal that seals the lid when the lid is in a sealed position and directs fluid runoff unimpeded back into the basin when the lid is the open position. The flexible seal includes a base, a flap extending from the base, and a protuberance extending from the flap. The flexible seal may be configured such that when the lid is in the sealed position at least a portion of a lower surface of the flap sealingly contacts a rim of the basin. The flexible seal may further be configured such that when the lid is in the open position, at a hinge side of the lid at least a portion of a lower surface of the protuberance has a negative slope relative to horizontal in a direction toward an opening in the basin.

According to one aspect of the invention, a medical device processor, includes a basin having an opening for receiving therein a medical device for processing and a rim at the perimeter of the opening; a lid displaceable at a hinge side of the lid between an open position to expose the opening and a sealed position, wherein the lid in the open position is at an acute angle relative to the lid in the sealed position; and a flexible seal disposed at least at the hinge side of the lid. The flexible seal includes a base fixed to an underside of the lid, a flap extending from the base and having an upper surface and a lower surface, wherein the upper surface faces toward the underside of the lid, and a protuberance extending from the flap and having an upper surface and a lower surface, wherein the upper surface faces toward the underside of the lid. The flexible seal is configured such that when the lid is in the sealed position at least a portion of the lower surface of the flap sealingly contacts the rim of the basin, and such that when the lid is in the open position, at the hinge side of the lid at least a portion of the lower surface of the protuberance has a negative slope relative to horizontal in a direction toward the opening in the basin.

Embodiments of the invention may include one or more of the following additional features separately or in combination.

The lid may be angularly displaceable about a hinge axis at the hinge side of the lid between the open position and a closed position, wherein in the closed position the lid covers the opening, and the lid may be further displaceable between the closed position and the sealed position.

The lid may be vertically displaceable between the closed position and the sealed position.

The flexible seal may be configured such that when the lid is in the closed position the protuberance is spaced from the rim of the basin.

The flap may be configured to flex relative to the base such that when the lid is displaced from the closed position to the sealed position the rim of the basin urges the flap and the protuberance closer to the underside of the lid.

The flap may be configured to slide along the rim of the basin as the lid is displaced from the closed position to the sealed position.

The rim may have a negative slope relative to horizontal in the direction toward the opening in the basin.

The flexible seal and the rim may both be annular in shape.

The lower surface of the flap may have an outer surface portion and an inner surface portion.

The inner surface portion may be disposed at an oblique angle relative to the outer surface portion.

The outer surface portion may include the portion of the lower surface of the flap that sealingly contacts the rim of the basin when the lid is in the sealed position.

The lid may be angularly displaceable about a hinge axis at the hinge side of the lid between the open position and a closed position, wherein in the closed position the lid covers the opening, and the lid may be further displaceable between the closed position and the sealed position, and the flap may be configured such that when the lid is in the closed position the outer surface portion faces away from the opening in the basin and the inner surface portion faces toward the opening in the basin.

The lid and the flexible seal may be configured such that when the lid is in a sealed position, a radially inward facing surface of the base, the upper surface of the flap, the inner surface portion of the lower surface of the flap, and the protuberance are exposed to decontaminant in the basin.

The medical device processor may include a decontaminant spray mechanism configured to spray decontaminant within the basin, and the decontaminant spray mechanism and the flap may be configured such that when the lid is in the sealed position, the decontaminant sprayed by the decontaminant spray mechanism contacts a radially inward facing surface of the base, the upper surface of the flap, the inner surface portion of the lower surface of the flap, and the protuberance.

The flexible seal may include an elastomeric material.

The elastomeric material may include a 40A durometer silicone rubber.

In side cross sectional view of the flexible seal the base may have a base width and a base length, and the flap may have a flap width and a flap length, wherein the flap width is less than the base width, and the flap length is greater than the base length.

In side cross sectional view of the flexible seal the protuberance may have a protuberance width and a protuberance length, and the flap may have a flap width and a flap length, wherein the protuberance width is less than the flap width, and the protuberance length is less than the flap length.

The flexible seal may include an outer lip that extends from the base and is separated from the flap by a groove.

The outer lip may be configured such that when the lid is in the sealed position the outer lip is in contact with the rim of the basin with the groove between the outer lip and the flap.

According to another aspect of the invention, a method of processing a medical device in a medical device processor is provided. The medical device processor may include a basin having an opening and a rim at the perimeter of the opening, a lid, and a flexible seal, the flexible seal including a base, a flap extending from the base, and a protuberance extending from the flap. The method may include inserting a medical device through the opening and into an interior of the basin; displacing the lid at a hinge side of the lid from an open position to a sealed position, wherein the lid in the open position is at an acute angle relative to the lid in the sealed position; while moving the lid to the sealed position, urging at least a portion of a lower surface of the flap of the flexible seal into sealing contact with the rim of the basin to create a seal between the lid and the rim to seal the interior of the basin from the exterior of the processor; conducting a decontamination cycle including imparting decontaminant against exposed surfaces of the flexible seal facing the interior of the basin; displacing the lid at the hinge side from the sealed position to the open position, wherein when the lid is in the open position, at the hinge side of the lid at least a portion of a lower surface of the protuberance has a negative slope relative to horizontal in a direction toward the opening in the basin; and directing fluid runoff from the lid to the protuberance of the flexible seal but away from the portion of the lower surface of the protuberance having the negative slope relative to horizontal, and vertically downward from the protuberance unimpeded back into the interior of the basin.

Embodiments of the invention may include one or more of the following additional features separately or in combination.

The directing the fluid runoff may include beading the fluid runoff at a distal end of the protuberance before directing the fluid runoff vertically downward.

In the sealed position the flexible seal may contact the rim of the basin to form a sealing interface, and the directing the fluid runoff may include preventing the fluid runoff from contacting a surface radially outward from the sealing interface.

The following description and the annexed drawings set forth certain illustrative embodiments of the invention. These embodiments are indicative, however, of but a few of the various ways in which the principles of the invention may be employed. Other objects, advantages and novel features according to aspects of the invention will become apparent from the following detailed description when considered in conjunction with the drawings.

### Brief Description of the Drawings

The annexed drawings, which are not necessarily to scale, show various aspects of the invention.
Fig. 1 is a perspective view of a medical device processor according to an embodiment of the invention, showing a lid thereof in an open position to show a flexible seal.
Fig. 2 is a side elevational view of the Fig. 1 processor, showing the lid in the open position.
Fig. 3 is a side elevational view of the Fig. 1 processor, except showing the lid in a closed position.
Fig. 4 is a side elevational view of the Fig. 1 processor, except showing the lid in a sealed position.
Fig. 5 is an enlarged side cross sectional view of the lid and the flexible seal at a hinge side of the lid and the Fig. 1 processor, showing the lid in the open position such as shown in Fig. 2.
Fig. 6 is an enlarged side cross sectional view of the lid, the flexible seal, and a basin rim at the hinge side of the lid and the Fig. 1 processor, showing the lid in the closed position such as shown in Fig. 3.
Fig. 7 is an enlarged side cross sectional view of the lid, the flexible seal, and the basin rim at the hinge side of the lid and the Fig. 1 processor, showing the lid in a partially compressed position.
Fig. 8 is an enlarged side cross sectional view of the lid, the flexible seal, and the basin rim at the hinge side of the lid and the Fig. 1 processor, showing the lid in another partially compressed position.
Fig. 9 is an enlarged side cross sectional view of the lid, the flexible seal, and the basin rim at the hinge side of the lid and the Fig. 1 processor, showing the lid in the sealed position such as shown in Fig. 4.
Fig. 10 is an enlarged side cross sectional view of the lid and the flexible seal at the hinge side of the lid and the Fig. 1 processor, showing the lid in an open position, similar to Fig. 5, and showing the path of fluid runoff after a processing cycle of the medical device processor.
Fig. 11 is a load deflection curve representing a load and deflection of the flexible seal.
Fig. 12 shows a flowchart of a method in accordance with an embodiment of the invention.

### Detailed Description

While the present invention can take many different forms, for the purpose of promoting an understanding of the principles of the invention, reference will now be made to the embodiments illustrated in the drawings and specific language will be used to describe the same. It will nevertheless be understood that no limitation of the scope of the invention is thereby intended. Any alterations and further modifications of the described embodiments, and any further applications of the principles of the invention as described herein, are contemplated as would normally occur to one skilled in the art to which the invention relates.

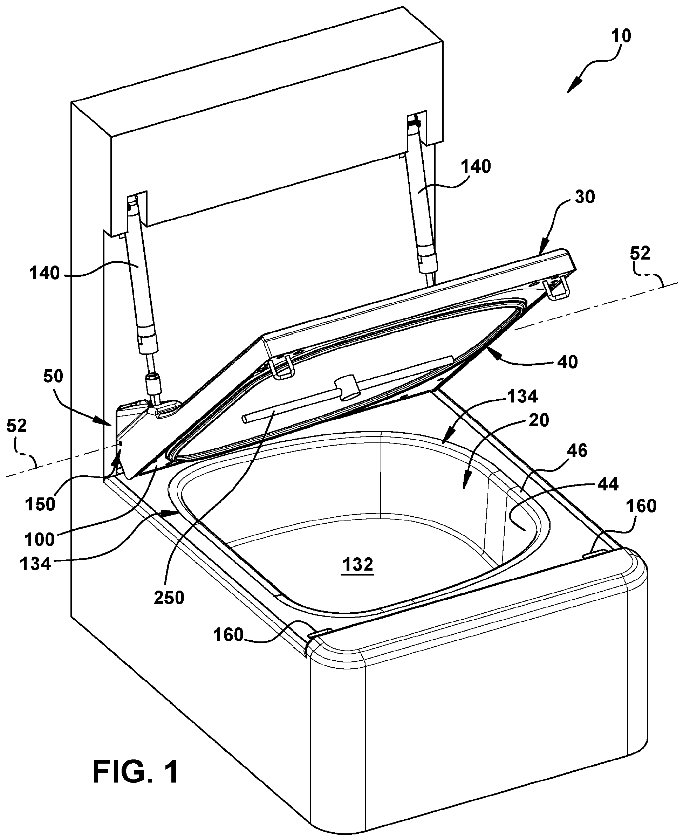

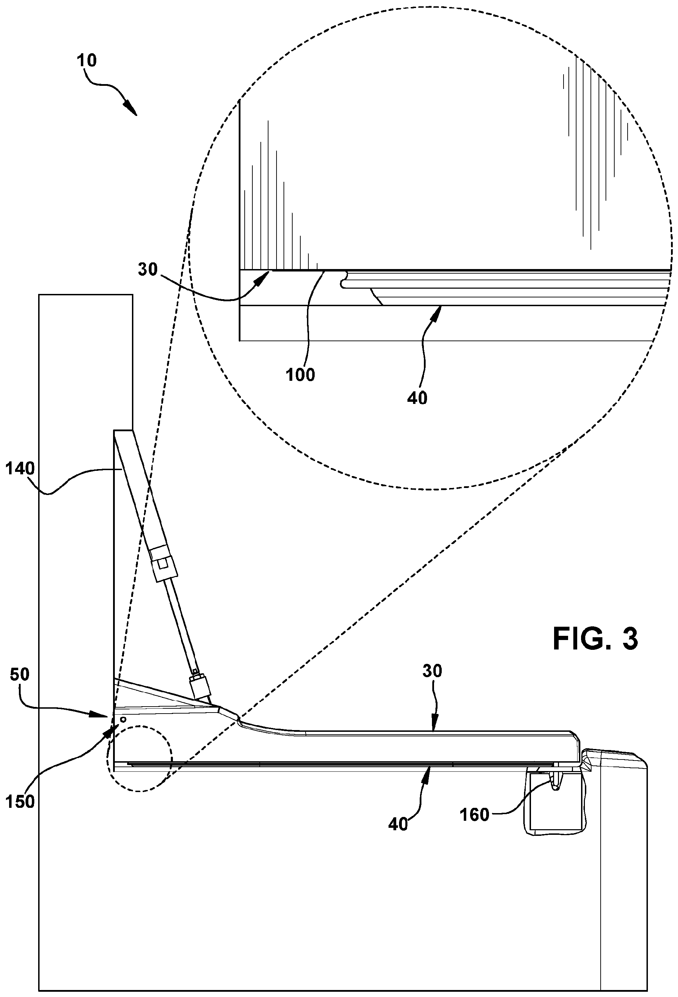

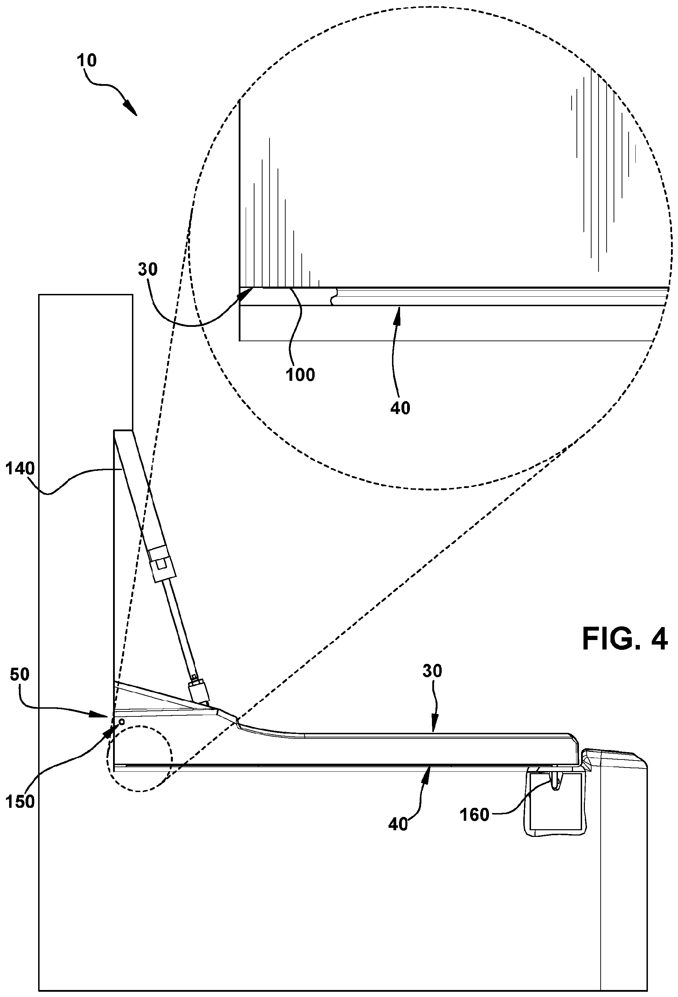

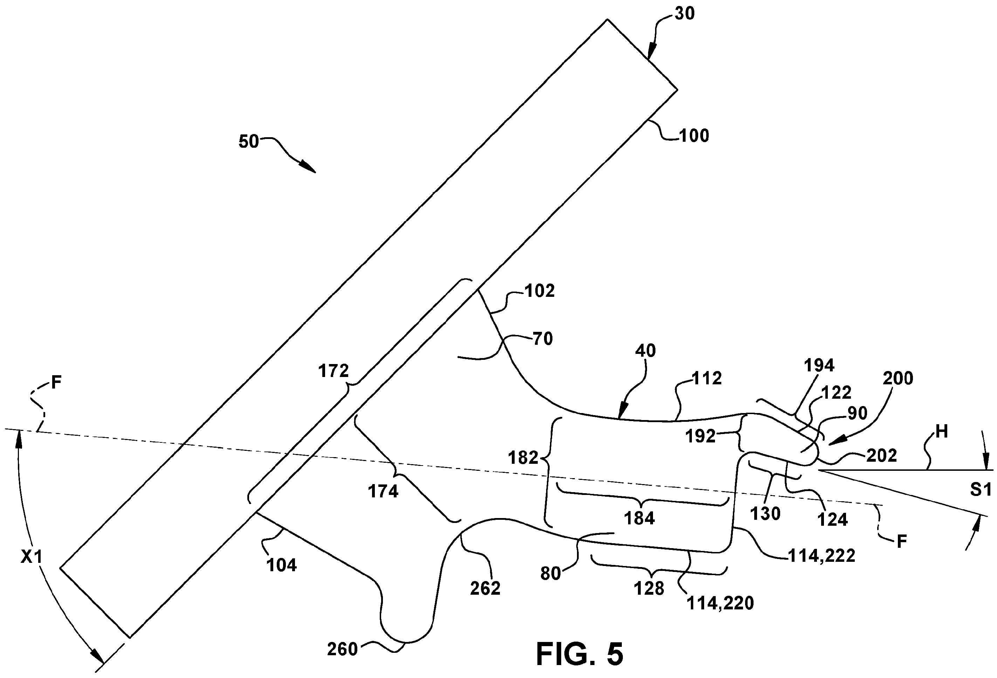

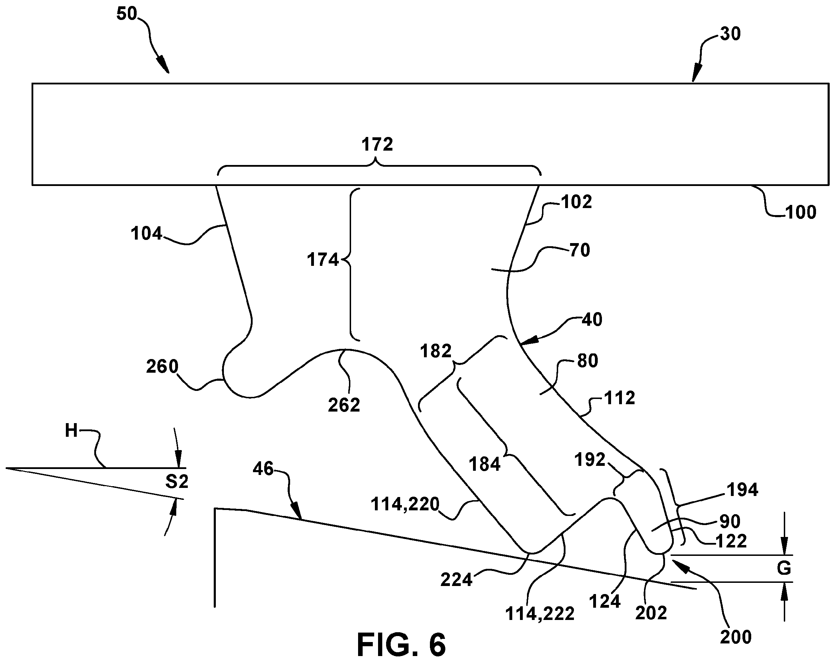

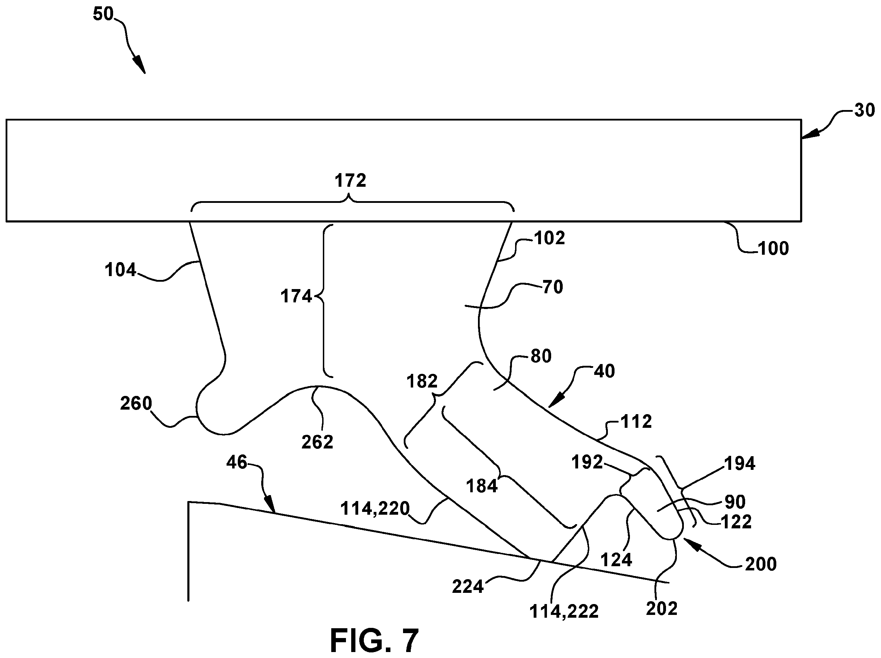

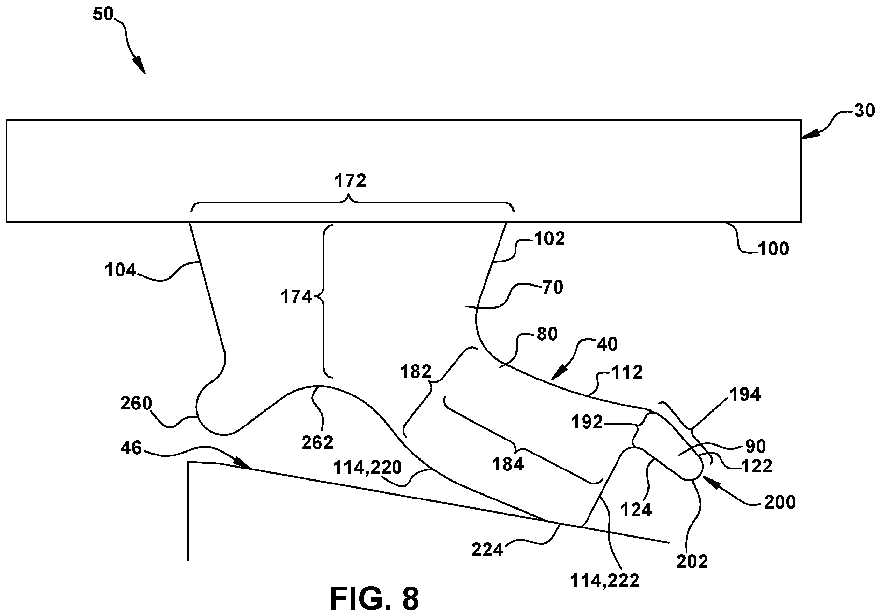

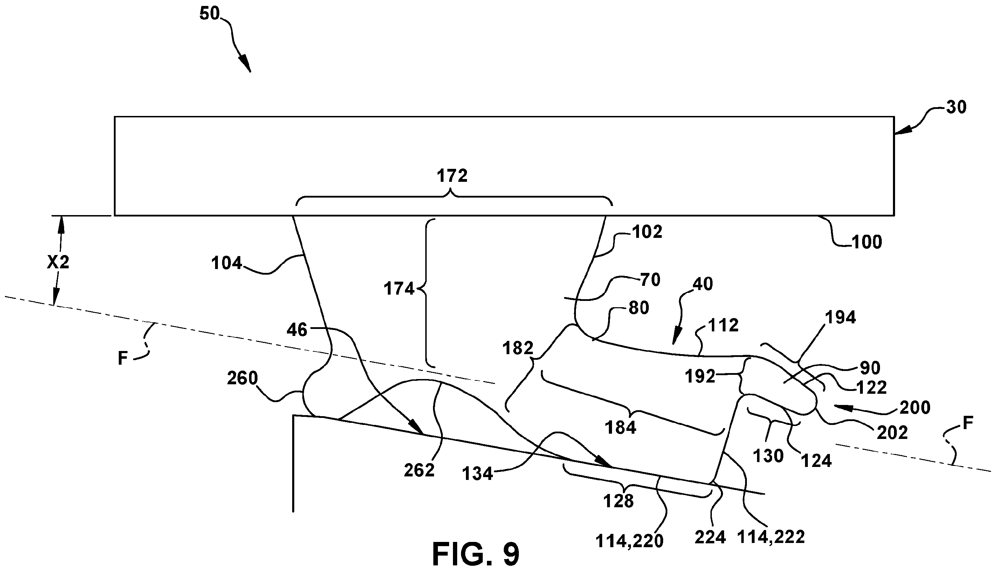

Figs. 1-10 show a medical device processor 10 in accordance with an embodiment of the invention. The medical device processor 10 may be any type of system for decontaminating medical devices or instruments, for example, by a sterilization process or a disinfection process. The medical device processor 10 includes a basin 20, a lid 30, and a flexible seal 40. The basin 20 has an opening 44 for receiving therein a medical device for processing and a rim 46 at the perimeter of the opening 44. The lid 30 is displaceable at a hinge side 50 of the lid 30 between an open position to expose the opening 44, as shown in Figs. 1, 2 and 5, and a sealed position, which is shown for example in Figs. 4 and 9, wherein the lid 30 in the open position is at an acute angle relative to the lid 30 in the sealed position. In some embodiments, the lid 30 may be angularly displaceable about a hinge axis 52 at the hinge side 50 between the open position and a closed position such as shown in Figs. 3 and 6, wherein in the closed position the lid 30 covers the opening 44, and further displaceable between the closed position and the sealed position. The flexible seal 40 is disposed at least at the hinge side 50 of the lid 30, and includes a base 70, a flap 80, and a protuberance 90. The base 70 is fixed to an underside 100 of the lid 30 and has a radially inward facing surface 102 and a radially outward facing surface 104. The flap 80 extends from the base 70 and has an upper surface 112 and a lower surface 114, with the upper surface 112 facing toward the underside 100 of the lid 30. The protuberance 90 extends from the flap 80 and has an upper surface 122 and a lower surface 124, with the upper surface 122 facing toward the underside 100 of the lid 30. The flexible seal 40 is configured such that when the lid 30 is in the sealed position (Fig. 9) at least a portion 128 of the lower surface 114 of the flap 80 sealingly contacts the rim 46 of the basin 20, and such that when the lid 30 is in the open position (Fig. 5), at the hinge side 50 of the lid 30 at least a portion 130 of the lower surface 124 of the protuberance 90 has a negative slope S1 relative to horizontal H in a direction toward the opening 44 in the basin 20. In some embodiments, the flexible seal 40 may also be configured such that when the lid 30 is in the closed position (Fig. 6), the protuberance 90 is spaced from the rim 46 of the basin 20 by a gap G.

As will be described in greater detail below, the flexible seal 40 advantageously provides a sealing interface 134 (Figs. 1 and 9) by means of its contact with the basin rim 46 to seal the basin 20 from the exterior of the processor 10 when the lid 30 is sealed during a processing cycle. The flexible seal 40 also advantageously provides a flexible shield that prevents lid droplet runoff from contacting any non-decontaminated surfaces such as the surface of the basin rim 46 radially outward from the sealing interface 134 when the lid 30 is angularly displaced, or hinged open, after a processing cycle. Because the protuberance 90 of the flexible seal 40 is exposed to an interior 132 of the basin 20 during a processing cycle, the interior facing portions of the flexible seal 40 and the basin 20 are fully decontaminated after a cycle is completed. Moreover, because the protuberance 90 directs the lid runoff across the decontaminated surfaces of the flexible seal 40 and unimpeded back into the basin 20, no additional drainage channel system is required on the basin 20. Still further, in embodiments where the gap G is provided between the protuberance 90 and the rim 46 of the basin 20 when the lid 30 is in the closed position, the gap G aids in preventing the protuberance 90 from contacting non-decontaminated surfaces such as the surface of the basin rim 46 radially outward from the sealing interface 134 when the lid 30 and more specifically the flexible seal 40 are uncompressed after a processing cycle, and thereby maintains the decontamination of the protuberance 90.

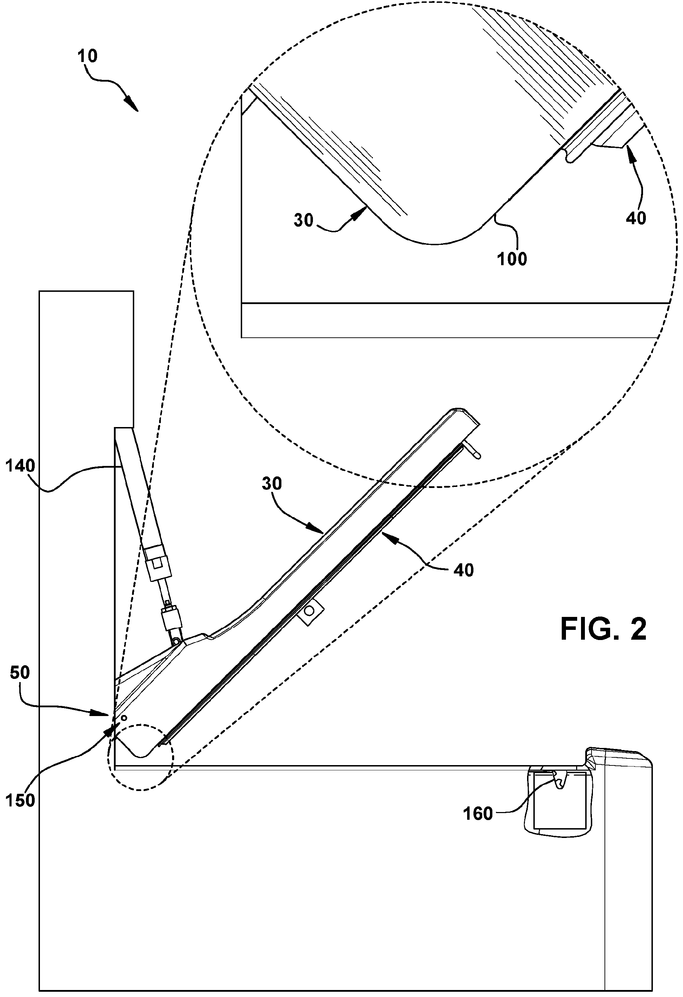

Turning initially then to Fig. 1, the medical device processor 10 is shown in perspective view with the lid 30 in an open position. Figs. 2-4 show side views of the lid 30 respectively in the open position, the closed position, and the sealed position. In the open position, the lid 30 is at an acute angle, or about 45 degrees in Figs. 1 and 2, relative to the position of the lid 30 when in the closed position (Fig. 3) and relative to the position of the lid 30 when in the sealed position (Fig. 4). One or more stops may be provided to prevent opening of the lid 30 beyond the acute angle. In the closed position, the lid 30 is generally parallel to horizontal H. In the sealed position, the lid 30 is also generally parallel to horizontal H but vertically displaced relative to the closed position. As will be appreciated, in some embodiments, vertical displacement between the closed position and sealed position may be omitted; in other words, in the sealed position the lid is also in the closed position. As used herein, horizontal H means the plane of the horizon, and vertical means at a right angle, or perpendicular, to the horizontal H or horizon.

In the illustrative embodiment, the lid 30 is configured for both angular displacement between the open position and the closed position as well as vertical displacement between the closed position and the sealed position. In some embodiments, the lid 30 may be displaced between the open position and the sealed position without an intermediate closed position and/or without a vertical displacement between the closed position and the sealed position. A mechanism or combination of mechanisms may be used to angularly displace the lid 30 and, if a vertical displacement capability is to be included, to vertically displace the lid 30. In the illustrative embodiment, a pair of rear mounted cylinder actuators 140 are configured to urge the lid 30 about a rear mounted hinge 150, or angularly displace the lid 30, with the hinge 150 defining the afore mentioned hinge axis 52. The rear mounted cylinder actuators 140 also work in conjunction with a pair of front mounted latch actuators 160 to vertically displace the lid 30.

In operation, when in the open position shown in Figs. 1 and 2, medical devices such as an endoscope can be inserted into the basin 20 of the medical device processor 10. To close the lid 30, the rear mounted cylinder actuators 140 angularly displace, or pivot, the lid 30 about the hinge axis 52 from the open position shown in Figs. 1 and 2 to the closed position shown in Fig. 3. Prior to starting a processing cycle, also referred to herein as a reprocessing cycle, the rear mounted cylinder actuators 140 and the front mounted latch actuators 160 vertically displace the lid 30 between the closed position shown in Fig. 3 to the sealed position shown in Fig. 4. In the sealed position, the flexible seal 40 creates a seal at the rim 46 of the basin 20 of the processor 10 to seal the interior 132 of the basin 20 from the exterior of the processor 10. A processing cycle can then be performed by the medical device processor 10. Once the processing cycle is completed, the order of operation is merely reversed. Thus, to unseal the lid 30, the actuators 140, 160 vertically displace the lid 30 from the sealed position shown in Fig. 4 to the closed position shown in Fig. 3. To open the lid 30, the rear mounted cylinder actuators 140 angularly displace the lid 30 from the closed position shown in Fig. 3 to the open position shown in Figs. 1 and 2. The decontaminated devices can then be removed from the basin 20.

Still referring to Fig. 1 and additionally to Figs. 6-9, the rim 46 of the medical device processor 10 is annular in shape and surrounds the opening 44 in the basin 20. The rim 46 is generally flat, or planar, in side cross sectional view. As shown in Fig. 6, in the illustrative embodiment, the rim 46 has a negative slope S2 relative to horizontal H in the direction toward the opening 44 in the basin 20. It will be appreciated that the rim 46 need not be sloped relative to horizontal H, or may be sloped in one arc portion of the rim 46 and not sloped in another arc portion of the rim 46, with the flexible seal 40 being sloped or contoured to match that of the rim 46 to ensure sealing contact with the rim 46.

The flexible seal 40 is annular in shape and, as shown in Fig. 1, generally matches the annular shape of the rim 46. The illustrative flexible seal 40 has a constant cross sectional area in side cross sectional view. Thus, the cross sectional area of the flexible seal 40 shown in Figs. 5-10 is continuous throughout the annular structure of the flexible seal 40. The invention contemplates, however, that the flexible seal 40, or characteristics of the flexible seal 40, may be disposed at the hinge side 50 of the lid 30 while a different type of seal or different seal characteristics may be disposed at non-hinge portions of the lid 30. Thus, the structure of a seal at the hinge side of the lid 30 may have a cross sectional area such as shown in Figs. 5-10, that is the flexible seal 40, whereas the structure of the seal at a non-hinge side of the lid 30 may have a different cross sectional area from that shown in Figs. 5-10. For example, an annular seal such as shown in Fig. 1 may include a portion having the characteristics of the flexible seal 40 disposed at the hinge side of the lid 30 and another portion having characteristics different from that of the flexible seal 40 disposed at the non-hinge side of the lid 30. In this way, that is with the flexible seal 40 being located at least at the hinge side of the lid 30, the flexible seal 40 provides the advantageous effects with respect to runoff described herein while the other non-hinge side portions of the seal provide a seal with the interface at the rim 46 of the processor 10. It will be appreciated that the runoff exhibited by the lid 30 usually will run down the lid 30 from the non-hinge side to the hinge side 50 of the lid 30 so that the flexible seal 40 usually need merely be disposed at the hinge side 50 of the lid 30 to redirect the runoff. On the other hand, cost considerations and ease of manufacture considerations may make a uniform cross section flexible seal more suitable or desirable.

The flexible seal 40 may be secured to the underside 100 of the lid 30 by an adhesive, for example, a cyanoacrylate. The flexible seal 40 is configured such that when the lid 30 is moved from the closed position to the sealed position, the flexible seal 40 is flexed and compressed by the rim 46 as the flap 80 of the flexible seal 40 slides along the rim 46. The flex and compression in the flexible seal 40 creates a seal at the interface of the flexible seal 40 and the rim 46, which in effect seals the interior 132 of the basin 20 from the exterior of the processor 10. To aid in the flexibility and compressibility of the flexible seal 40, the flexible seal 40 may be made of an elastomeric material. In one form, the flexible seal 40 may be made of a 40A durometer silicone rubber. To aid the flap 80 in sliding along the rim 46 and maintaining a tight seal, the flexible seal 40 may be treated with a low friction surface treatment such as fluorination.

Figs. 5-10 show the flexible seal 40 and an exemplary size and geometry of its components the base 70, the flap 80, and the protuberance 90 relative to one another. The base 70 has a base width 172 and a base length 174; the flap 80 has a flap width 182 and a flap length 184; and the protuberance 90 has a protuberance width 192 and a protuberance length 194. The base width 172 includes the width of the base 70 at the connection of the base 70 to the underside 100 of the lid 30 and the width of the base 70 at the location where the flap 80 extends from the base 70. In the illustrative embodiment, the base width 172 is tapered as the base 70 extends from the underside 100 of the lid 30 to the flap 80. The base length 174 is the length of the base 70 from the location of the connection of the base 70 to the underside of the lid 30 to the location where the flap 80 extends from the base 70. The flap width 182 includes the width of the flap 80 at the location where the flap 80 extends from the base 70 and the width of the flap 80 at the location where the protuberance 90 extends from the flap 80. In the illustrative embodiment, the flap width 182 remains substantially constant, that is the flap width 182 does not deviate more than five percent (5%) in width as the flap 80 extends from the base 70 to the protuberance 90. The flap length 184 is the length of the flap 80 from the location where the flap 80 extends from the base 70 to the location where the protuberance 90 extends from the flap 80. The protuberance width 192 includes the width of the protuberance 90 at the location where the protuberance 90 extends from the flap 80 and the width of the protuberance 90 at the location where the protuberance 90 transitions to a curved tip portion 200. In the illustrative embodiment, the protuberance width 192 is tapered as the protuberance 90 extends from the flap 80 to the curved tip portion 200. The protuberance length 194 is the length of the protuberance 90 from the location where the protuberance 90 extends from the flap 80 to the farthest location from flap 80, that is, to a distal end 202 of the curved tip portion 200 of the protuberance 90.

In the illustrative embodiment, the flap width 182 is less than the base width 172. Further, the flap length 184 is greater than the base length 174. Also in the illustrative embodiment, the protuberance width 192 is less than the flap width 182. In addition, the protuberance length 194 is less than the flap length 184. The inventor has found that any one or more of the foregoing relative dimensions in the base 70, the flap 80, and the protuberance 90 may aid in realizing the advantageous effects of the invention.

Still referring to Figs. 5-10, the flexible and compressible characteristics of the base 70, the flap 80, and the protuberance 90 that make up the flexible seal 40 will now be described. The flap 80 is configured to flex relative to the base 70 such that when the lid 30 is displaced from the closed position shown in Figs. 3 and 6 to the sealed position shown in Figs. 4 and 9, the rim 46 of the basin 20 urges, as by bending, the flap 80 and the protuberance 90 closer to the underside 100 of the lid 30. To illustrate this further, reference is made to the lower surface 114 of the flap 80, which in the illustrative embodiment has an outer surface portion 220 and an inner surface portion 222, where the inner surface portion 222 is disposed at an oblique angle, at right angles in the illustrative embodiment, relative to the outer surface portion 220. As shown in Fig. 9, the outer surface portion 220 includes the portion 128 of the lower surface 114 of the flap 80 that sealingly contacts the rim 46 of the basin 20 when the lid 30 is in the sealed position. As shown from Fig. 6 to Fig. 9, as the flap 80 flexes closer to the underside 100 of the lid 30, a rounded corner portion 224 at the junction of the inner surface portion 220 and the outer surface portion 222 slides along the rim 46 of the basin 20 until the portion 128 of the lower surface 112 of the flap 80 sealingly contacts the rim 46 of the basin 20 (Fig. 9). The inner surface portion 222 meanwhile faces toward the opening 44 in the basin 20 when the lid 30 is in the sealed position. As shown in Figs. 3 and 6, the illustrative flap 80 is also configured such that when the lid 30 is in the closed position the outer surface portion 220 faces away from the opening 44 in the basin 20 and the inner surface portion 222 faces toward the opening 44 in the basin 20.

The flexibility in the flexible seal 40 may further be demonstrated by a comparison of Figs. 5 and 9. In Fig. 5, the lid 30 is in the open position and the flexible seal 40 is in an unflexed state. In Fig. 5, a central axis F of the flap 80 is at an angle X1 relative to the underside 100 of the lid 30. **In** Fig. 9, the lid 30 is in the sealed position and the flexible seal 40 is in a fully flexed state. In Fig. 9, the central axis F of the flap 80 is at an angle X2 relative to the underside 100 of the lid 30. It will be appreciated, then, that the angle of flex of the flap 80 is about equal to X1 minus X2, which in the illustrative embodiment is about 50 degrees minus 20 degrees, yielding about a 30 degree flex. Thus, the change in angle of the center axis F of the flap 80 relative to the underside 100 of the lid 30 as the lid 30 is urged from the closed position shown in Figs. 3 and 6 to the sealed position shown in Figs. 4 and 9 is about 30 degrees. In some embodiments, the flexibility may be greater or less depending on the material of the flexible seal 40 and associated bending and flex characteristics, and/or the desired sealing characteristics.

The flexibility in the flexible seal 40 enables surfaces of the flexible seal 40 facing inward toward the interior 132 of the basin 20 to be exposed to decontaminant during a processing cycle, for example, by immersion, spray, or condensation. As shown in Fig. 9, the surfaces of the flexible seal 40 facing inward toward the interior 132 of the basin 20 include the radially inward facing surface 102 of the base 70, the upper surface 112 of the flap 80, the inner surface portion 222 of the lower surface 114 of the flap 80, and the protuberance 90. During a processing cycle, decontaminant is imparted on these surfaces for example by static immersion, that is, when the basin 20 is filled or nearly filled with decontaminant. When the lid 30 is in the sealed position shown in Figs. 4 and 9, the decontaminant contacts the radially inward facing surface 102 of the base 70, the upper surface 112 of the flap 80, the inner surface portion 222 of the lower surface 114 of the flap 80, and the protuberance 90.

In other embodiments, the decontaminant may be imparted on the inward facing surfaces of the flexible seal 40 by additional or alternative means. As illustrated in Fig. 1, for example, the medical device processor 10 may include a decontaminant spray mechanism 250 for spraying a decontaminant within the basin 20 including to the underside of the lid 30 and to the inward facing surfaces of the flexible seal 40. In the illustrative embodiment, the decontaminant spray mechanism 250 is positioned such that when the lid 30 is in the sealed position shown in Figs. 4 and 9, the decontaminant sprayed by the decontaminant spray mechanism 250 contacts the radially inward facing surface 102 of the base 70, the upper surface 112 of the flap 80, the inner surface portion 222 of the lower surface 114 of the flap 80, and the protuberance 90. As will be appreciated, the illustrative flexible seal 40 lends itself well to being imparted with decontaminant during a processing cycle whether by static immersion, by means of a mechanism such as the decontaminant spray mechanism 250, by condensation, or by a combination of these means or other means.

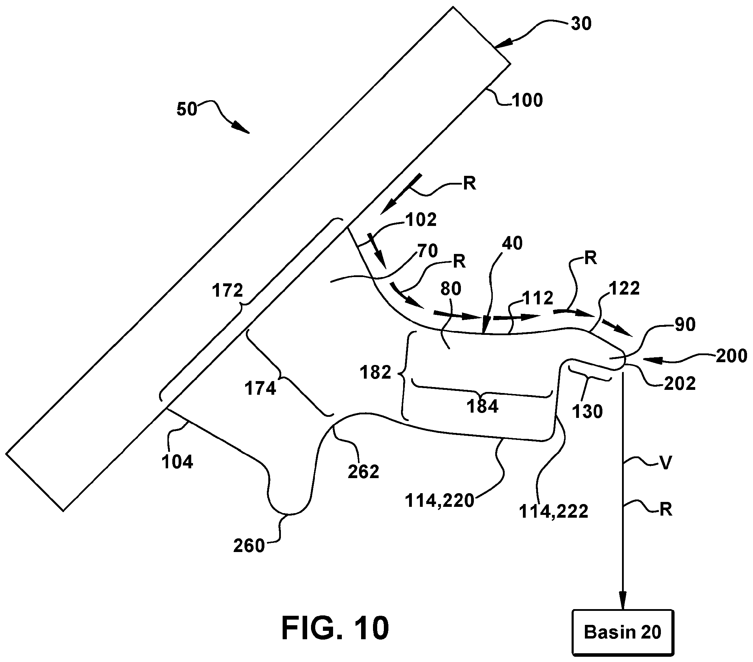

When a processing cycle is completed and the lid 30 is opened, the flexible seal 40 returns to its unflexed state, where it then directs fluid droplets running down the lid 30 unimpeded back into the interior 132 of the basin 20. Fig. 10 shows the lid 30 in the open position after a processing cycle, at the acute angle of 45 degrees relative to the horizontal H. The path of fluid runoff is represented by arrows R in Fig. 10. As shown in Fig. 10, and additionally referring again to Fig. 5, when the lid 30 is in the open position and the flexible seal 40 is in the unflexed state, the portion 130 of the lower surface 124 of the protuberance 90 has a negative slope S1 relative to horizontal H in a direction toward the opening 44 in the basin 20. The inventor found that this negative slope S1 aids in beading fluid runoff R at the distal end 202 of the protuberance 90 and thus enabling gravity to prevent the fluid runoff R from continuing to run across the protuberance 90 (to the left in Fig. 10) and potentially into contact with non-decontaminated surfaces such as the surface of the basin rim 46 radially outward from the sealing interface 134 between the flexible seal 40 and the basin rim 46. As shown in Fig. 10, the fluid runoff R runs along the underside 100 of the lid 30, the radially inward facing surface 102 of the base 70 of the flexible seal 40, the upper surface 112 of the flap 80 of the flexible seal 40, the upper surface 122 of the protuberance 90 of the flexible seal 40, and downward along a vertical line V projecting downward from the distal end 202 of the protuberance 90 and into the basin 20. Notably, the fluid runoff R does not run along the lower surface 124 of the protuberance 90 but instead, owing to the negative slope S1 of the portion 130 of the lower surface 124, is pulled by gravity at the distal end 202 of the protuberance 90.

Referring briefly to Figs. 5, 6 and 10, in embodiments where a gap G is desired to be maintained between the protuberance 90 and the rim 46 of the basin 20 when the lid 30 is in the closed position, it will be appreciated that, on the one hand, the length 194 of the protuberance 90 is short enough to maintain the gap G and, on the other hand, the length of the portion 130 of the lower surface 124 of the protuberance 90 is long enough to ensure the fluid runoff R beads at the distal end 202 of the protuberance 90 sufficiently to be pulled down by gravity. The inventor has found that the relative size and geometries of the base 70, the flap 80, and the protuberance 90 described herein and shown in the figures provides an effective balance in maintaining the gap G while providing sufficient beading at the distal end 202 of the protuberance 90 to enable gravity-driven fluid runoff R. In some embodiments, the protuberance 90 may be lengthened so long as the gap G, if necessary or desired, is still met. Conversely, the protuberance 90 may be shortened so long as the negative slope S1 of the portion 130 of the lower surface 124 of the protuberance 90 still results in sufficient beading at the distal end 202 of the protuberance 90 to enable gravity to pull the fluid runoff R vertically V down to the interior 132 of the basin 20.

The compressibility of the flexible seal 40 may also be demonstrated by a comparison of Figs. 5 and 9. As shown in Fig. 5, the illustrative flexible seal 40 additionally includes an outer lip 260 that extends from the base 70 and is separated from the flap 80 by a groove 262. The outer lip 260 is configured such that when the lid 30 is in the sealed position (Fig. 9) the outer lip 260 is in contact with the rim 46 of the basin 20 with the groove 262 between the outer lip 260 and the flap 80. Configured in this way, the outer lip 260 takes up some of the compressive load on the flap 80 to prevent over flexing or over compression of the flap 80 when the lid 30 is urged from the closed position shown in Figs. 3 and 6 to the sealed position shown in Figs. 4 and 9. It will be appreciated, of course, that in applications where sealing the lid 30 is not expected to cause over flexing or over compression of the flap 80, the outer lip 260 may be omitted.

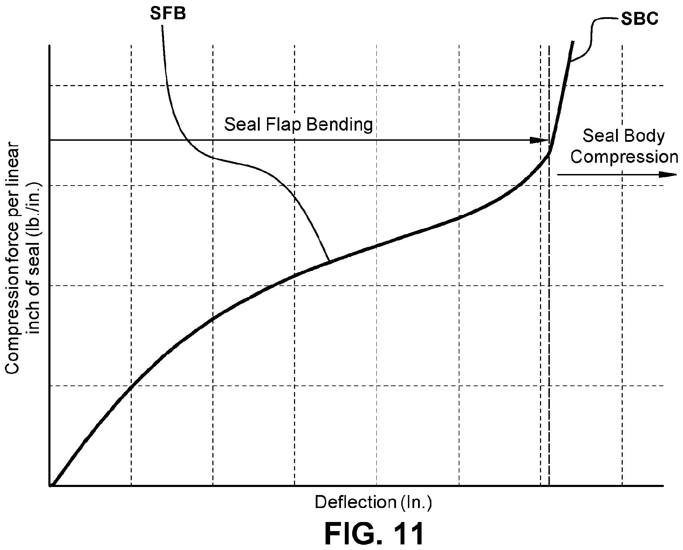

Referring now to Fig. 11, which shows a load deflection curve of the flexible seal 40, it can be seen that the flexible seal 40 has two stages, a seal flap 80 bending stage represented by the solid line SFB to the left of the vertical dashed line in Fig. 11, and a seal body 70 compression stage represented by the solid line SBC to the right of the vertical dashed line. The seal flap 80 bending stage is characterized by relatively high deformation and relatively low stiffness. The seal body 70 compression stage is characterized by relatively low deformation and relatively high stiffness. In Fig. 11, the x-axis, or independent axis, represents the flex or deflection of the flap 80 in inches, and the y-axis, or dependent axis, represents the compressive force per linear inch of the flexible seal 40.

The seal flap 80 bending stage occurs when the flap 80 is bending, flexing, or deflecting, as when the lid 30 is moved from the closed position shown in Figs. 3 and 6 to the sealed position shown in Figs. 4 and 9. As shown by the line SFB, in the seal flap 80 bending stage, the flap 80 exhibits a relatively high deformation or deflection and a relatively low stiffness, relative to the seal body 70 compression stage. Thus, the seal flap 80 bending stage is dominated by bending of the flap 80. As described above, the bending of the flap 80 aids in exposing the surfaces of the protuberance 90 to decontaminant during a processing cycle. Also, in the seal flap 80 bending stage, the afore described low friction surface treatment aids in enabling the flap 80 to slide along the sealing surface of the rim 46 of the basin 20 without sticking and thereby aiding in creating a tight seal with the rim 46. The tight seal prevents fluid egress from the interior 132 of the basin 20, for example, from static air pressure and/or spray impingement from the decontaminant spray mechanism 250. Additionally, the high deformation and low stiffness loading profile allows the flexible seal 40 to compensate for compression loading imbalances resulting from lid/basin flatness deviation and/or assembly tolerances along the length of the flexible seal 40, for example the flexible seal 40 having the annular shape length in Fig. 1, or a flexible seal having a length corresponding to the hinge side 50 of the lid 30, as described above, or other length.

The seal body 70 compression stage occurs when the flexible seal 40 is compressed, more specifically, after the flap 80 and the outer lip 260 of the flexible seal 40 have contacted the rim 46 of the basin 20 (Fig. 9) and the actuators 140, 160 continue to urge the flexible seal 40 against the rim 46. The flap 80 and the outer lip 260, if provided, transfer the compressive load to the base 70, which is also referred to herein as the body of the flexible seal 40. As shown by the line SBC in Fig. 11, in the seal body 70 compression stage, the base 70 of the flexible seal 40 exhibits a relatively low deformation or deflection and a relatively high stiffness, relative to the seal flap 80 bending stage. Thus, the seal body 70 compression stage is dominated by compression of the base 70 of the flexible seal 40. As will be appreciated, the seal body 70 compression stage aids in preventing the aforementioned over flexing or over compression of the flap 80 as the flap 80 contacts and slides against the rim 46 and is urged against the rim 46 to seal the lid 30 to the rim 46.

As will be appreciated, the flexible seal 40 has an integrated shielding feature in the protuberance 90. The base 70 of the flexible seal 40 is secured to the underside 100 of the lid 30 with an adhesive, such that the lower point of the flap 80 contacts the sealing surface of the rim 46 of the basin 20, and the protuberance 90 is facing inward toward the center of the basin 20. When the processing cycle begins, the lid 30 is compressed downwards, flexing the flap 80 such that the flap 80 creates the sealing interface 134 with the rim 46 sealing surface. This flexure of the flap 80 exposes the interior surfaces of the flexible seal 40 to decontaminant inside of the basin 20 during a processing cycle, namely the radially inward facing surface 102 of the base 70, the upper surface 112 of the flap 80, the inner surface portion 222 of the lower surface 114 of the flap 80, and the protuberance 90. When the processing cycle concludes, the compression force on the lid 30 is removed and the flexible seal 40 returns to its unflexed and uncompressed state as shown in Figs. 3 and 6. Finally, as the lid 30 hinges to its open state shown in Figs. 1 and 2, the fluid runoff from the lid 30 runs over the decontaminated interior surfaces 112, 222 and the protuberance 90, then unimpeded back into the basin 20, never contacting any non-decontaminated surfaces such as the surface of the basin rim 46 radially outward from the sealing interface 134 between the flexible seal 40 and the basin rim 46 (see Fig. 10).

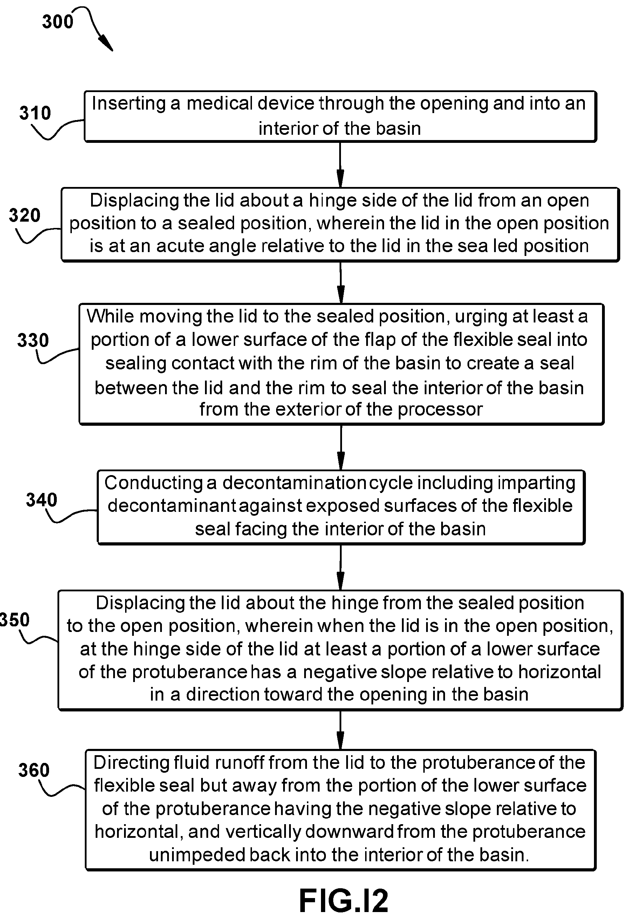

Referring now to Fig. 12, a flowchart 300 for a method of processing a medical device in a medical device processor, such as the medical device processor 10, is shown. Thus, the medical device processor may include a basin having an opening and a rim at the perimeter of the opening, a lid, and a flexible seal, the flexible seal including a base, a flap extending from the base, and a protuberance extending from the flap. Step 310 of the method may include inserting a medical device through the opening and into an interior of the basin. Step 320 may include displacing the lid at a hinge side of the lid from an open position to a sealed position, wherein the lid in the open position is at an acute angle relative to the lid in the sealed position. Step 330 may include, while moving the lid to the sealed position, urging at least a portion of a lower surface of the flap of the flexible seal into sealing contact with the rim of the basin to create a seal between the lid and the rim to seal the interior of the basin from the exterior of the processor. Step 340 may include conducting a decontamination cycle including imparting decontaminant against exposed surfaces of the flexible seal facing the interior of the basin. Step 350 may include displacing the lid at the hinge side from the sealed position to the open position, wherein when the lid is in the open position, at the hinge side of the lid at least a portion of a lower surface of the protuberance has a negative slope relative to horizontal in a direction toward the opening in the basin. Step 360 may include directing fluid runoff from the lid to the protuberance of the flexible seal but away from the portion of the lower surface of the protuberance having the negative slope relative to horizontal, and vertically downward from the protuberance unimpeded back into the interior of the basin.

The directing the fluid runoff may include beading the fluid runoff at a distal end of the protuberance before directing the fluid runoff vertically downward.

In the sealed position the flexible seal may contact the rim of the basin to form a sealing interface, and the directing the fluid runoff may include preventing the fluid runoff from contacting a surface radially outward from the sealing interface.

Although the invention has been shown and described with respect to a certain embodiment or embodiments, it is obvious that alterations and modifications will occur to others skilled in the art upon the reading and understanding of this specification and the annexed drawings, within the scope of the invention as defined by the appended claims. In particular regard to the various functions performed by the above described elements (components, assemblies, devices, compositions, etc.), the terms (including a reference to a "means") used to describe such elements are intended to correspond, unless otherwise indicated, to any element which performs the specified function of the described element (i.e., that is functionally equivalent), even though not structurally equivalent to the disclosed structure which performs the function in the herein illustrated exemplary embodiment or embodiments of the invention. In addition, while a particular feature of the invention may have been described above with respect to only one or more of several illustrated embodiments, such feature may be combined with one or more other features of the other embodiments, as may be desired and advantageous for any given or particular application.

## Claims

1. A medical device processor (10), comprising:
a basin (20) having an opening (44) for receiving therein a medical device for processing and a rim (46) at the perimeter of the opening (44);
a lid (30) displaceable at a hinge side (50) of the lid (30) between an open position to expose the opening (44) and a sealed position, wherein the lid (30) in the open position is at an acute angle relative to the lid (30) in the sealed position; and
a flexible seal (40) disposed at least at the hinge side (50) of the lid (30), the flexible seal (40) including
a base (70) fixed to an underside (100) of the lid (30),
a flap (80) extending from the base (70) and having an upper surface (112) and a lower surface (114), wherein the upper surface (112) faces toward the underside (100) of the lid (30), and
a protuberance (90) extending from the flap (80) and having an upper surface (122) and a lower surface (124), wherein the upper surface (122) faces toward the underside (100) of the lid (30),
wherein the flexible seal (40) is configured such that when the lid (30) is in the sealed position at least a portion (128) of the lower surface (114) of the flap (80) sealingly contacts the rim (46) of the basin (20), and such that when the lid (30) is in the open position, at the hinge side (50) of the lid (30) at least a portion (130) of the lower surface (124) of the protuberance (90) has a negative slope relative to horizontal in a direction toward the opening (44) in the basin (20).

2. The medical device processor (10) of claim 1, wherein the lid (30) is angularly displaceable about a hinge axis (52) at the hinge side (50) of the lid (30) between the open position and a closed position, wherein in the closed position the lid (30) covers the opening (44), and wherein the lid (30) is further displaceable between the closed position and the sealed position.

3. The medical device processor (10) of claim 2, wherein the lid (30) is vertically displaceable between the closed position and the sealed position.

4. The medical device processor (10) of claim 2, wherein the flexible seal (40) is configured such that when the lid (30) is in the closed position the protuberance (90) is spaced from the rim (46) of the basin (20).

5. The medical device processor (10) of claim 2, wherein the flap (80) is configured to flex relative to the base (70) such that when the lid (30) is displaced from the closed position to the sealed position the rim (46) of the basin (20) urges the flap (80) and the protuberance (90) closer to the underside (100) of the lid (30).

6. The medical device processor (10) of claim 2, wherein the flap (80) is configured to slide along the rim (46) of the basin (20) as the lid (30) is displaced from the closed position to the sealed position.

7. The medical device processor (10) of claim 1, wherein the rim (46) has a negative slope relative to horizontal in the direction toward the opening (44) in the basin (20).

8. The medical device processor (10) of claim 1, wherein the flexible seal (40) and the rim (46) are both annular in shape.

9. The medical device processor (10) of claim 1, wherein the lower surface (114) of the flap (80) has an outer surface portion (220) and an inner surface portion (222).

10. The medical device processor (10) of claim 9, wherein the inner surface portion (222) is disposed at an oblique angle relative to the outer surface portion (220).

11. The medical device processor (10) of claim 9, wherein the outer surface portion (220) includes the portion (128) of the lower surface (114) of the flap (80) that sealingly contacts the rim (46) of the basin (20) when the lid (30) is in the sealed position.

12. The medical device processor (10) of claim 9, wherein the lid (30) is angularly displaceable about a hinge axis (52) at the hinge side (50) of the lid (30) between the open position and a closed position, wherein in the closed position the lid (30) covers the opening (44), and wherein the lid (30) is further displaceable between the closed position and the sealed position, wherein the flap (80) is configured such that when the lid (30) is in the closed position the outer surface portion (220) faces away from the opening (44) in the basin (20) and the inner surface portion faces (222) toward the opening (44) in the basin (20).

13. The medical device processor (10) of claim 9, wherein the lid (30) and the flexible seal (40) are configured such that when the lid (30) is in a sealed position, a radially inward facing surface (102) of the base (70), the upper surface (112) of the flap (80), the inner surface portion (222) of the lower surface (114) of the flap (80), and the protuberance (90) are exposed to decontaminant in the basin (20).

14. The medical device processor (10) of claim 9, further comprising a decontaminant spray mechanism (250) configured to spray decontaminant within the basin (20), wherein the decontaminant spray mechanism (250) and the flap (80) are configured such that when the lid (30) is in the sealed position, the decontaminant sprayed by the decontaminant spray mechanism (250) contacts a radially inward facing surface (102) of the base (70), the upper surface (112) of the flap (80), the inner surface portion (222) of the lower surface (114) of the flap (80), and the protuberance (90).

15. The medical device processor (10) of claim 1, wherein the flexible seal (40) includes an elastomeric material.

16. The medical device processor (10) of claim 15, wherein the elastomeric material includes a 40A durometer silicone rubber.

17. The medical device processor (10) of claim 1, wherein in side cross sectional view of the flexible seal (40) the base (70) has a base width (172) and a base length (174), and the flap (80) has a flap width (182) and a flap length (184), wherein the flap width (182) is less than the base width (172), and the flap length (184) is greater than the base length (174).

18. The medical device processor (10) of claim 1, wherein in side cross sectional view of the flexible seal (40) the protuberance (90) has a protuberance width (192) and a protuberance length (194), and the flap (80) has a flap width (182) and a flap length (184), wherein the protuberance width (192) is less than the flap width (182), and the protuberance length (194) is less than the flap length (184).

19. The medical device processor (10) of claim 1, wherein the flexible seal (40) includes an outer lip (260) that extends from the base (70) and is separated from the flap (80) by a groove (262).

20. The medical device processor (10) of claim 19, wherein the outer lip (260) is configured such that when the lid (30) is in the sealed position the outer lip (260) is in contact with the rim (46) of the basin (20) with the groove (262) between the outer lip (260) and the flap (80).

21. A method of processing a medical device in a medical device processor (10) including a basin (20) having an opening (44) and a rim (46) at the perimeter of the opening (44), a lid (30), and a flexible seal (40), the flexible seal (40) including a base (70), a flap (80) extending from the base (70), and a protuberance (90) extending from the flap (80), the method comprising:
inserting a medical device through the opening (44) and into an interior (132) of the basin (20);
displacing the lid (30) at a hinge side (50) of the lid (30) from an open position to a sealed position, wherein the lid (30) in the open position is at an acute angle relative to the lid (30) in the sealed position;
while moving the lid (30) to the sealed position, urging at least a portion (128) of a lower surface (114) of the flap (80) of the flexible seal (40) into sealing contact with the rim (46) of the basin (20) to create a seal between the lid (30) and the rim (46) to seal the interior (132) of the basin (20) from the exterior of the processor (10);
conducting a decontamination cycle including imparting decontaminant against exposed surfaces of the flexible seal (40) facing the interior (132) of the basin (20);
displacing the lid (30) at the hinge side (50) of the lid (30) from the sealed position to the open position, wherein when the lid (30) is in the open position, at the hinge side (50) of the lid (30) at least a portion (130) of a lower surface (124) of the protuberance (90) has a negative slope relative to horizontal in a direction toward the opening (44) in the basin (20); and
directing fluid runoff from the lid (30) to the protuberance (90) of the flexible seal (40) but away from the portion (130) of the lower surface (124) of the protuberance (90) having the negative slope relative to horizontal, and vertically downward from the protuberance (90) unimpeded back into the interior (132) of the basin (20).

22. The method of claim 21, wherein the directing the fluid runoff includes beading the fluid runoff at a distal end (202) of the protuberance (90) before directing the fluid runoff vertically downward.

23. The method of claim 21, wherein in the sealed position the flexible seal (40) contacts the rim (46) of the basin (20) to form a sealing interface (134), and wherein the directing the fluid runoff includes preventing the fluid runoff from contacting a surface radially outward from the sealing interface (134).

## Patentansprüche

1. Prozessor (10) medizinischer Vorrichtungen, umfassend:
ein Becken (20), das eine Öffnung (44), um darin eine medizinische Vorrichtung zur Verarbeitung aufzunehmen, und einen Rand (46) am Umfang der Öffnung (44) aufweist;
einen Deckel (30), der an einer Scharnierseite (50) des Deckels (30) zwischen einer offenen Position, um die Öffnung (44) freizulegen, und einer abgedichteten Position verschiebbar ist,
wobei der Deckel (30) in der offenen Position in einem spitzen Winkel relativ zum Deckel (30) in der abgedichteten Position steht; und
eine flexible Dichtung (40), die mindestens an der Scharnierseite (50) des Deckels (30) angeordnet ist, wobei die flexible Dichtung (40) Folgendes beinhaltet:
eine Basis (70), die an einer Unterseite (100) des Deckels (30) befestigt ist,
eine Klappe (80), die sich von der Basis (70) erstreckt und eine obere Fläche (112) und eine untere Fläche (114) aufweist, wobei die obere Fläche (112) der Unterseite (100) des Deckels (30) zugewandt ist, und
einen Vorsprung (90), der sich von der Klappe (80) erstreckt und eine obere Fläche (122) und eine untere Fläche (124) aufweist, wobei die obere Fläche (122) der Unterseite (100) des Deckels (30) zugewandt ist,
wobei die flexible Dichtung (40) derart konfiguriert ist, dass, wenn der Deckel (30) in der abgedichteten Position ist, mindestens ein Abschnitt (128) der unteren Fläche (114) der Klappe (80) abdichtend den Rand (46) des Beckens (20) kontaktiert, und derart, dass, wenn der Deckel (30) in der offenen Position ist, an der Scharnierseite (50) des Deckels (30) mindestens ein Abschnitt (130) der unteren Fläche (124) des Vorsprungs (90) eine negative Neigung relativ zur Horizontalen in einer Richtung zur Öffnung (44) im Becken (20) aufweist.

2. Prozessor (10) medizinischer Vorrichtungen nach Anspruch 1, wobei der Deckel (30) um eine Scharnierachse (52) an der Scharnierseite (50) des Deckels (30) zwischen der offenen Position und einer geschlossenen Position winkelmäßig verschiebbar ist, wobei der Deckel (30) in der geschlossenen Position die Öffnung (44) abdeckt und wobei der Deckel (30) ferner zwischen der geschlossenen Position und der abgedichteten Position verschiebbar ist.

3. Prozessor (10) medizinischer Vorrichtungen nach Anspruch 2, wobei der Deckel (30) vertikal zwischen der geschlossenen Position und der abgedichteten Position verschiebbar ist.

4. Prozessor (10) medizinischer Vorrichtungen nach Anspruch 2, wobei die flexible Dichtung (40) derart konfiguriert ist, dass, wenn der Deckel (30) in der geschlossenen Position ist, der Vorsprung (90) vom Rand (46) des Beckens (20) beabstandet ist.

5. Prozessor (10) medizinischer Vorrichtungen nach Anspruch 2, wobei die Klappe (80) dazu konfiguriert ist, sich relativ zur Basis (70) zu biegen, derart, dass, wenn der Deckel (30) aus der geschlossenen Position in die abgedichtete Position verschoben wird, der Rand (46) des Beckens (20) die Klappe (80) und den Vorsprung (90) näher an die Unterseite (100) des Deckels (30) drückt.

6. Prozessor (10) medizinischer Vorrichtungen nach Anspruch 2, wobei die Klappe (80) dazu konfiguriert ist, entlang des Rands (46) des Beckens (20) zu gleiten, wenn der Deckel (30) aus der geschlossenen Position in die abgedichtete Position verschoben wird.

7. Prozessor (10) medizinischer Vorrichtungen nach Anspruch 1, wobei der Rand (46) eine negative Neigung relativ zur Horizontalen in Richtung der Öffnung (44) im Becken (20) aufweist.

8. Prozessor (10) medizinischer Vorrichtungen nach Anspruch 1, wobei die flexible Dichtung (40) und der Rand (46) beide ringförmig sind.

9. Prozessor (10) medizinischer Vorrichtungen nach Anspruch 1, wobei die untere Fläche (114) der Klappe (80) einen äußeren Flächenabschnitt (220) und einen inneren Flächenabschnitt (222) aufweist.

10. Prozessor (10) medizinischer Vorrichtungen nach Anspruch 9, wobei der innere Flächenabschnitt (222) in einem schrägen Winkel relativ zum äußeren Flächenabschnitt (220) angeordnet ist.

11. Prozessor (10) medizinischer Vorrichtungen nach Anspruch 9, wobei der äußere Flächenabschnitt (220) den Abschnitt (128) der unteren Fläche (114) der Klappe (80) beinhaltet, der abdichtend den Rand (46) des Beckens (20) kontaktiert, wenn der Deckel (30) in der abgedichteten Position ist.

12. Prozessor (10) medizinischer Vorrichtungen nach Anspruch 9, wobei der Deckel (30) um eine Scharnierachse (52) an der Scharnierseite (50) des Deckels (30) zwischen der offenen Position und einer geschlossenen Position winkelmäßig verschiebbar ist, wobei der Deckel (30) in der geschlossenen Position die Öffnung (44) abdeckt und wobei der Deckel (30) ferner zwischen der geschlossenen Position und der abgedichteten Position verschiebbar ist, wobei die Klappe (80) derart konfiguriert ist, dass, wenn der Deckel (30) in der geschlossenen Position ist, der äußere Flächenabschnitt (220) von der Öffnung (44) im Becken (20) abgewandt ist und der innere Flächenabschnitt (222) der Öffnung (44) im Becken (20) zugewandt ist.

13. Prozessor (10) medizinischer Vorrichtungen nach Anspruch 9, wobei der Deckel (30) und die flexible Dichtung (40) derart konfiguriert sind, dass, wenn der Deckel (30) in einer abgedichteten Position ist, eine radial nach innen gewandte Fläche (102) der Basis (70), die obere Fläche (112) der Klappe (80), der innere Flächenabschnitt (222) der unteren Fläche (114) der Klappe (80) und der Vorsprung (90) einem Dekontaminationsmittel im Becken (20) ausgesetzt sind.

14. Prozessor (10) medizinischer Vorrichtungen nach Anspruch 9, ferner umfassend einen Dekontaminationsmittelsprühmechanismus (250), der dazu konfiguriert ist, ein Dekontaminationsmittel innerhalb des Beckens (20) zu versprühen, wobei der Dekontaminationsmittelsprühmechanismus (250) und die Klappe (80) derart konfiguriert sind, dass, wenn der Deckel (30) in der abgedichteten Position ist, das durch den Dekontaminationsmittelsprühmechanismus versprühte Dekontaminationsmittel (250) eine radial nach innen gewandte Fläche (102) der Basis (70), die obere Fläche (112) der Klappe (80), den inneren Flächenabschnitt (222) der unteren Fläche (114) der Klappe (80) und den Vorsprung (90) kontaktiert.

15. Prozessor (10) medizinischer Vorrichtungen nach Anspruch 1, wobei die flexible Dichtung (40) ein Elastomermaterial beinhaltet.

16. Prozessor (10) medizinischer Vorrichtungen nach Anspruch 15, wobei das Elastomermaterial einen Silikonkautschuk mit einer Härte von 40A beinhaltet.

17. Prozessor (10) medizinischer Vorrichtungen nach Anspruch 1, wobei in der Seitenquerschnittsansicht der flexiblen Dichtung (40) die Basis (70) eine Basisbreite (172) und eine Basislänge (174) aufweist und die Klappe (80) eine Klappenbreite (182) und eine Klappenlänge (184) aufweist, wobei die Klappenbreite (182) kleiner ist als die Basisbreite (172) und die Klappenlänge (184) größer ist als die Basislänge (174).

18. Prozessor (10) medizinischer Vorrichtungen nach Anspruch 1, wobei in der Seitenquerschnittsansicht der flexiblen Dichtung (40) der Vorsprung (90) eine Vorsprungsbreite (192) und eine Vorsprungslänge (194) aufweist und die Klappe (80) eine Klappenbreite (182) und eine Klappenlänge (184) aufweist, wobei die Vorsprungsbreite (192) kleiner ist als die Klappenbreite (182) und die Vorsprungslänge (194) kleiner ist als die Klappenlänge (184).

19. Prozessor (10) medizinischer Vorrichtungen nach Anspruch 1, wobei die flexible Dichtung (40) eine äußere Lippe (260) beinhaltet, die sich von der Basis (70) erstreckt und von der Klappe (80) durch eine Rille (262) getrennt ist.

20. Prozessor (10) medizinischer Vorrichtungen nach Anspruch 19, wobei die äußere Lippe (260) derart konfiguriert ist, dass, wenn der Deckel (30) in der abgedichteten Position ist, die äußere Lippe (260) in Kontakt mit dem Rand (46) des Beckens (20) steht, wobei sich die Rille (262) zwischen der äußeren Lippe (260) und der Klappe (80) befindet.

21. Verfahren zum Verarbeiten einer medizinischen Vorrichtung in einem Prozessor (10) medizinischer Vorrichtungen, der ein Becken (20), das eine Öffnung (44) und einen Rand (46) am Umfang der Öffnung (44) aufweist, einen Deckel (30) und eine flexible Dichtung (40) beinhaltet, wobei die flexible Dichtung (40) eine Basis (70), eine sich von der Basis (70) erstreckende Klappe (80) und einen sich von der Klappe (80) erstreckenden Vorsprung (90) beinhaltet, wobei das Verfahren Folgendes umfasst:
Einlegen einer medizinischen Vorrichtung durch die Öffnung (44) und in einen Innenraum (132) des Beckens (20);
Verschieben des Deckels (30) an einer Scharnierseite (50) des Deckels (30) aus einer offenen Position in eine abgedichtete Position, wobei der Deckel (30) in der offenen Position in einem spitzen Winkel relativ zum Deckel (30) in der abgedichteten Position steht;
während des Bewegens des Deckels (30) in die abgedichtete Position, Drücken mindestens eines Abschnitts (128) einer unteren Fläche (114) der Klappe (80) der flexiblen Dichtung (40) in abdichtenden Kontakt mit dem Rand (46) des Beckens (20), um eine Dichtung zwischen dem Deckel (30) und dem Rand (46) zu erzeugen, um den Innenraum (132) des Beckens (20) von der Außenseite des Prozessors (10) abzudichten;
Durchführen eines Dekontaminationszyklus, der ein Aufbringen eines Dekontaminationsmittels auf freiliegende Flächen der flexiblen Dichtung (40) beinhaltet, die dem Innenraum (132) des Beckens (20) zugewandt sind;
Verschieben des Deckels (30) an der Scharnierseite (50) des Deckels (30) aus der abgedichteten Position in die offene Position, wobei, wenn der Deckel (30) in der offenen Position ist, an der Scharnierseite (50) des Deckels (30) mindestens ein Abschnitt (130) einer unteren Fläche (124) des Vorsprungs (90) eine negative Neigung relativ zur Horizontalen in einer Richtung zur Öffnung (44) im Becken (20) aufweist; und
Leiten eines Fluidablaufs vom Deckel (30) zum Vorsprung (90) der flexiblen Dichtung (40), aber weg vom Abschnitt (130) der unteren Fläche (124) des Vorsprungs (90), der die negative Neigung relativ zur Horizontalen aufweist, und vertikal nach unten vom Vorsprung (90) ungehindert zurück in den Innenraum (132) des Beckens (20).

22. Verfahren nach Anspruch 21, wobei das Leiten des Fluidablaufs ein Abperlen des Fluidablaufs an einem distalen Ende (202) des Vorsprungs (90) beinhaltet, bevor der Fluidablauf vertikal nach unten geleitet wird.

23. Verfahren nach Anspruch 21, wobei die flexible Dichtung (40) in der abgedichteten Position den Rand (46) des Beckens (20) kontaktiert, um eine Dichtungsgrenzfläche (134) zu bilden, und wobei das Leiten des Fluidablaufs beinhaltet, dass der Fluidablauf daran gehindert wird, eine Fläche zu kontaktieren, die sich radial außerhalb der Dichtungsgrenzfläche (134) befindet.

## Revendications

1. Processeur de dispositif médical (10), comprenant :
une cuve (20) présentant une ouverture (44) destinée à recevoir en son sein un dispositif médical à traiter et un rebord (46) au périmètre de l'ouverture (44) ;
un couvercle (30) déplaçable, au niveau d'un côté charnière (50) du couvercle (30), entre une position ouverte pour exposer l'ouverture (44) et une position étanche, dans lequel le couvercle (30) en position ouverte se trouve à un angle aigu par rapport au couvercle (30) en position étanche ; et
un joint flexible (40) disposé au moins au niveau du côté charnière (50) du couvercle (30), le joint flexible (40) comportant
une base (70) fixée à une face inférieure (100) du couvercle (30),
une languette (80) s'étendant à partir de la base (70) et présentant une surface supérieure (112) et une surface inférieure (114), dans lequel la surface supérieure (112) est orientée vers la face inférieure (100) du couvercle (30), et
une protubérance (90) s'étendant à partir de la languette (80) et présentant une surface supérieure (122) et une surface inférieure (124), dans lequel la surface supérieure (122) est orientée vers la face inférieure (100) du couvercle (30),
dans lequel le joint flexible (40) est agencé de sorte que, lorsque le couvercle (30) se trouve en position étanche, au moins une partie (128) de la surface inférieure (114) de la languette (80) entre en contact d'étanchéité avec le rebord (46) de la cuve (20), et de sorte que, lorsque le couvercle (30) se trouve en position ouverte, au niveau du côté charnière (50) du couvercle (30), au moins une partie (130) de la surface inférieure (124) de la protubérance (90) présente une pente négative par rapport à l'horizontale dans une direction vers l'ouverture (44) dans la cuve (20).

2. Processeur de dispositif médical (10) selon la revendication 1, dans lequel le couvercle (30) est déplaçable angulairement autour d'un axe de charnière (52), au niveau du côté charnière (50) du couvercle (30), entre la position ouverte et une position fermée, dans lequel, en position fermée, le couvercle (30) recouvre l'ouverture (44), et dans lequel le couvercle (30) est également déplaçable entre la position fermée et la position étanche.

3. Processeur de dispositif médical (10) selon la revendication 2, dans lequel le couvercle (30) est déplaçable verticalement entre la position fermée et la position étanche.

4. Processeur de dispositif médical (10) selon la revendication 2, dans lequel le joint flexible (40) est agencé de sorte que, lorsque le couvercle (30) se trouve en position fermée, la protubérance (90) est espacée du rebord (46) de la cuve (20).

5. Processeur de dispositif médical (10) selon la revendication 2, dans lequel la languette (80) est agencée pour fléchir par rapport à la base (70) de sorte que, lorsque le couvercle (30) est déplacé de la position fermée à la position étanche, le rebord (46) de la cuve (20) sollicite la languette (80) et la protubérance (90) et les rapproche de la face inférieure (100) du couvercle (30).

6. Processeur de dispositif médical (10) selon la revendication 2, dans lequel la languette (80) est agencée pour glisser le long du rebord (46) de la cuve (20) à mesure que le couvercle (30) est déplacé de la position fermée à la position étanche.

7. Processeur de dispositif médical (10) selon la revendication 1, dans lequel le rebord (46) présente une pente négative par rapport à l'horizontale dans la direction vers l'ouverture (44) dans la cuve (20).

8. Processeur de dispositif médical (10) selon la revendication 1, dans lequel le joint flexible (40) et le rebord (46) sont tous deux de forme annulaire.

9. Processeur de dispositif médical (10) selon la revendication 1, dans lequel la surface inférieure (114) de la languette (80) présente une partie de surface extérieure (220) et une partie de surface intérieure (222).

10. Processeur de dispositif médical (10) selon la revendication 9, dans lequel la partie de surface intérieure (222) est disposée à un angle oblique par rapport à la partie de surface extérieure (220).

11. Processeur de dispositif médical (10) selon la revendication 9, dans lequel la partie de surface extérieure (220) comporte la partie (128) de la surface inférieure (114) de la languette (80) qui entre en contact d'étanchéité avec le rebord (46) de la cuve (20) lorsque le couvercle (30) se trouve en position étanche.

12. Processeur de dispositif médical (10) selon la revendication 9, dans lequel le couvercle (30) est déplaçable angulairement autour d'un axe de charnière (52), au niveau du côté charnière (50) du couvercle (30), entre la position ouverte et une position fermée, dans lequel, en position fermée, le couvercle (30) recouvre l'ouverture (44), et dans lequel le couvercle (30) est également déplaçable entre la position fermée et la position étanche, dans lequel la languette (80) est agencée de sorte que, lorsque le couvercle (30) se trouve en position fermée, la partie de surface extérieure (220) est orientée à l'opposé de l'ouverture (44) dans la cuve (20) et la partie de surface intérieure (222) est orientée vers l'ouverture (44) dans la cuve (20).

13. Processeur de dispositif médical (10) selon la revendication 9, dans lequel le couvercle (30) et le joint flexible (40) sont agencés de sorte que, lorsque le couvercle (30) se trouve en position étanche, une surface (102) de la base (70) orientée radialement vers l'intérieur, la surface supérieure (112) de la languette (80), la partie de surface intérieure (222) de la surface inférieure (114) de la languette (80), et la protubérance (90) sont exposées à du décontaminant dans la cuve (20).

14. Processeur de dispositif médical (10) selon la revendication 9, comprenant en outre un mécanisme de pulvérisation de décontaminant (250) agencé pour pulvériser du décontaminant à l'intérieur de la cuve (20), dans lequel le mécanisme de pulvérisation de décontaminant (250) et la languette (80) sont agencés de sorte que, lorsque le couvercle (30) se trouve en position étanche, le décontaminant pulvérisé par le mécanisme de pulvérisation de décontaminant (250) entre en contact avec une surface (102) de la base (70) orientée radialement vers l'intérieur, la surface supérieure (112) de la languette (80), la partie de surface intérieure (222) de la surface inférieure (114) de la languette (80), et la protubérance (90).

15. Processeur de dispositif médical (10) selon la revendication 1, dans lequel le joint flexible (40) comporte un matériau élastomère.

16. Processeur de dispositif médical (10) selon la revendication 15, dans lequel le matériau élastomère comporte un caoutchouc de silicone de dureté 40A (duromètre).

17. Processeur de dispositif médical (10) selon la revendication 1, dans lequel, en vue en coupe transversale latérale du joint flexible (40), la base (70) présente une largeur de base (172) et une longueur de base (174), et la languette (80) présente une largeur de languette (182) et une longueur de languette (184), dans lequel la largeur de languette (182) est inférieure à la largeur de base (172), et dans lequel la longueur de languette (184) est supérieure à la longueur de base (174).

18. Processeur de dispositif médical (10) selon la revendication 1, dans lequel, en vue en coupe transversale latérale du joint flexible (40), la protubérance (90) présente une largeur de protubérance (192) et une longueur de protubérance (194), et la languette (80) présente une largeur de languette (182) et une longueur de languette (184), dans lequel la largeur de protubérance (192) est inférieure à la largeur de languette (182), et dans lequel la longueur de protubérance (194) est inférieure à la longueur de languette (184).

19. Processeur de dispositif médical (10) selon la revendication 1, dans lequel le joint flexible (40) comporte une lèvre extérieure (260) qui s'étend à partir de la base (70) et est séparée de la languette (80) par une rainure (262).

20. Processeur de dispositif médical (10) selon la revendication 19, dans lequel la lèvre extérieure (260) est agencée de sorte que, lorsque le couvercle (30) se trouve en position étanche, la lèvre extérieure (260) est en contact avec le rebord (46) de la cuve (20), la rainure (262) se trouvant entre la lèvre extérieure (260) et la languette (80).

21. Un procédé de traitement d'un dispositif médical dans un processeur de dispositif médical (10) comportant une cuve (20) comportant une ouverture (44) et un rebord (46) au périmètre de l'ouverture (44), un couvercle (30), et un joint flexible (40), le joint flexible (40) comportant une base (70), une languette (80) s'étendant à partir de la base (70), et une protubérance (90) s'étendant à partir de la languette (80), le procédé comprenant :
l'insertion d'un dispositif médical à travers l'ouverture (44) et dans un intérieur (132) de la cuve (20) ;
le déplacement du couvercle (30), au niveau d'un côté charnière (50) du couvercle (30), d'une position ouverte à une position étanche, dans lequel le couvercle (30) en position ouverte se trouve à un angle aigu par rapport au couvercle (30) en position étanche ;
la sollicitation, pendant le déplacement du couvercle (30) vers la position étanche, d'au moins une partie (128) d'une surface inférieure (114) de la languette (80) du joint flexible (40) pour la mettre en contact d'étanchéité avec le rebord (46) de la cuve (20) afin de créer une étanchéité entre le couvercle (30) et le rebord (46) afin d'étancher l'intérieur (132) de la cuve (20) par rapport à l'extérieur du processeur (10) ;
la réalisation d'un cycle de décontamination comportant l'application de décontaminant contre des surfaces exposées du joint flexible (40) orientées vers l'intérieur (132) de la cuve (20) ;
le déplacement du couvercle (30), au niveau du côté charnière (50) du couvercle (30), de la position étanche à la position ouverte, dans lequel, lorsque le couvercle (30) se trouve en position ouverte, au niveau du côté charnière (50) du couvercle (30), au moins une partie (130) d'une surface inférieure (124) de la protubérance (90) présente une pente négative par rapport à l'horizontale dans une direction vers l'ouverture (44) dans la cuve (20) ; et
l'orientation d'un ruissellement de fluide depuis le couvercle (30) vers la protubérance (90) du joint flexible (40) mais à l'écart de la partie (130) de la surface inférieure (124) de la protubérance (90) présentant la pente négative par rapport à l'horizontale, et verticalement vers le bas depuis la protubérance (90), sans entrave, de retour dans l'intérieur (132) de la cuve (20).

22. Procédé selon la revendication 21, dans lequel l'orientation du ruissellement de fluide comporte la formation en gouttelettes du ruissellement de fluide à une extrémité distale (202) de la protubérance (90) avant l'orientation du ruissellement de fluide verticalement vers le bas.

23. Procédé selon la revendication 21, dans lequel, en position étanche, le joint flexible (40) entre en contact avec le rebord (46) de la cuve (20) pour former une interface d'étanchéité (134), et dans lequel l'orientation du ruissellement de fluide comporte la prévention du contact du ruissellement de fluide avec une surface située radialement à l'extérieur de l'interface d'étanchéité (134).
